# EUROPEAN PATENT SPECIFICATION

(11) **EP 3 041 803 B1**
(45) Date of publication and mention of the grant of the patent: **27.11.2019**
(21) Application number: 15712274.8
(22) Date of filing: 12.03.2015
(51) Int. Cl.: C03C 17/00, A61M 5/178, A61J 1/06, A61J 1/20, C03C 17/22, C03C 17/23, C03C 17/30, C03C 17/32

(54) **METHOD OF HANDLING A LIQUID DRUG FORMULATION**
VERFAHREN ZUR HANDHABUNG EINER FLÜSSIGEN ARZNEIMITTELFORMULIERUNG
PROCÉDÉ DE MANIPULATION D'UNE FORMULATION DE MÉDICAMENT LIQUIDE

(30) Priority: 13.03.2014 US 201461952450 P
(43) Date of publication of application: 13.07.2016
(73) Proprietor: STEVANATO GROUP INTERNATIONAL A. S., 844 03 Bratislava (SK)
(72) Inventor: BUREAU, Christophe, F-38410 Uriage (FR)
(74) Representative: Mitola, Marco
(86) International application number: PCT/EP2015/055169
(87) International publication number: WO 2015/136037

(56) References cited:
- WO-A1-99/44755
- WO-A2-2014/008138
- Michael Bonewitz: "Pharmaceutical Packaging", , 31 December 2008 (2008-12-31), XP055196222, Retrieved from the Internet: URL:http://www.schott.com/magazine/german/ download/sol108_01_coatings.pdf [retrieved on 2015-06-16]
- Syringe: "PACKAGING Autumn 2012 Volume 4 Issue 4 92 INTERNATIONAL PHARMACEUTICAL INDUSTRY", , 3 December 2012 (2012-12-03), XP055196214, Retrieved from the Internet: URL:http://ipimediaworld.com/wp-content/up loads/2012/12/1-Syringe-Siliconisation-Tre nds-Methods-Analysis-Procedures.pdf [retrieved on 2015-06-16]

## Description

The present invention relates to a method, as described in the attached claims, of handling a prefilled liquid drug formulation.

The stability of liquid pharmaceutical drug formulations - in particular over storage - is central to the efficacy and non toxicity of said drug formulation, as it is administered to the patient, for example by injection, infusion or inhalation.

Any liquid contained in any solid primary container will interact with it, extract components from it, and hence be modified: the longer the formulation and primary container are in contact, the more this interaction has time to produce its effects, and potentially create non anticipated problems. Many stability studies taking place during the development of pharmaceutical drug formulations aim at merely determining whether this level of interaction between the formulation and its primary container is acceptable for the targeted safety and efficacy levels.

This type of interaction may take place regardless of the actual nature of the drug formulation and of the active ingredient in it. But it is a fact that less stability issues have happened in the past with drugs that were produced by chemistry, in particular because chemical drugs had to be isolated - which supposes a minimum degree of stability.

Since the 80's, more and more drugs are being derived from biotechnologies, and in particular from recombinant proteins.

Recombinant therapeutic proteins are of a complex nature (composed of a long chain of amino acids, modified amino acids, derivatized by sugar moieties, folded by complex mechanisms). These proteins are made in living cells (bacteria, yeast, animal or human cell lines). The ultimate characteristics of a drug containing a recombinant therapeutic protein are to a large part determined by the process through which they are produced: choice of the cell type, development of the genetically modified cell for production, production process, purification process, formulation of the therapeutic protein into a drug.

After the expiry of the patent of approved recombinant drugs (e.g. insulin, human growth hormone, interferons, erythropoietin, monoclonal antibodies (mAbs), Fusion proteins and more) any other biotech company can "copy" and market these biologics (thus called biosimilars).

The key advantage of biotech drugs is their strong selectivity for a given set of molecular targets, which aims at obtaining a better drug efficacy while producing less side effects. These drugs are being intensively studied for the treatment of chronic diseases such as diabetes, rheumatoid arthritis, multiple sclerosis, various cancers, .etc.

Another common point of biotech drugs is their fragility relative to chemical drugs: as organic molecules, biotech and small chemical drugs have in common an intrinsic sensitivity of their functional groups (primary structure) to e.g. oxidation, hydrolysis,...etc, or of their intramolecular bonds between vicinal functional groups (secondary structure). However, due to their large molecular size, biotech drugs have specific internal 3 dimensional arrangements in space, or so called tertiary structure held by hydrogen bonding networks, that are the source of their function (the usual image of the "key and locker"). Sometimes, biotech drug molecules tend to self assemble, building quaternary structures held by ionic bridges or hydrogen bridges. These tertiary and quaternary structures are key to the very function of the biotech drugs, and are held by bonds of smaller energy than the covalent and vicinal hydrogen bonds holding primary and secondary structures: this explains why - while all drugs can show instability upon interacting with a primary container - biotech drugs may show higher intrinsic sensitivity. This explains also why the issue of drug/container interactions is being presently re-examined, as more and more biotech drugs are being developed by the pharmaceutical industry, in response to the high level trend of personalized medicine in particular.

One of the unsolved issues in the development of biotech drugs is the change of primary container taking place at a late stage, when the formulation has been validated in a given form, but requires modifications to be compatible with the final primary container in which it will be commercialized. Coating the inner surface of pharmaceutical glass containers is known, among other, from the following documents: "PACKAGING Autumn 2012 Volume 4 Issue 4 92 INTERNATIONAL PHARMACEUTICAL INDUSTRY" (3 December 2012, XP055196214); WO2014/008138 A2; and WO99/44755 A1. However, these documents do not address in details the problems linked to the change of primary container.

At early stages, drugs are being formulated in vials, i.e. small glass containers with a rubber cap. This container format is now a standard in the pharmaceutical industry, as it is the format compatible with sample holders used in the various analytical machines used for the various studies of said early stage formulations, through a so-called "combinatorial" approach. In this step, formulations are "optimized" for their stability: various additives are included in the formulation in order to optimize the stability of the resulting formulation, e.g. by measuring the quantity of particles generated over time in the solution and revealing an undesired aggregation of the protein, or by measuring the tertiary and quaternary structure of the protein by circular dichroism...etc. The optimal formulation is obtained through a so called "design of experiment" (DOE) in which the number of trial formulations is minimized and yet delivers the formulation having the optimal stability.

Since - as mentioned before - any liquid interacts with any solid container, it is not apparent that this formulation having the optimal stability also integrates any potential instability stemming from the primary container - a glass vial at that stage.

Formulations thus optimized then undergo various levels of test to establish their therapeutic interest. The first step, a preclinical phase, is to find a promising agent, which involves taking advantage of the advances made in understanding a disease, pharmacology, computer science, and chemistry. The next step before attempting a clinical trial in humans is to test the drug in living animals. The objectives of early in vivo testing are to demonstrate the safety of the proposed medication. If the safety of the proposed medication is shown, the stage is set for phase 1 of clinical trials comprising phases 1, 2 and 3. Phase 1 studies focus on the safety and pharmacology of a compound. Phase 2 studies examine the effectiveness of a compound. Phase 3 trials are to confirm previous findings in a larger population. If a drug survives the clinical trials, a written application is submitted containing all the preclinical and clinical information obtained during the testing phase to get approval for introduction of the drug into market. It is important to note that drug approval is given only on the formulation and container configuration used in Phase 3, which is thus also a "freezing step" in terms of final delivery mode.

Usually, all trials made between pre-clinical and Phase 2 (included) are performed with the same container format as the one that was optimized at research level, i.e. a vial made of medical grade type 1 glass and rubber cap. If, and only if, the drug formulation passes Phase 2 successfully, drug development teams consider new primary container formats for Phase 3, as this will "freeze" the format under which the drug formulation will be commercialized.

For a number of fairly well established reasons ranging from exactness of dosage or convenience to differentiation versus competition, more and more drugs are sold in prefilled format, i.e. in a "ready-to-use" format. For injectables, it can take the form of prefilled syringes or prefilled cartridges, while for inhalables, it can be e.g. in the form of prefilled cartridges.

The change of container taking place between Phase 2 and Phase 3 may be the source of new instability issues, hence requiring a re-optimization of the formulation.

Indeed, syringes and cartridges, while essentially in glass today, like vials, actually bear a lubricant to enable the gliding of the rubber plunger allowing to expel the drug out of the primary container. This means that while the drug formulation is in contact with essentially a glass surface in vials, it suddenly "sees" the surface of a silicone oil layer when poured into the final prefilled container.

Silicone oil and glass have, for example, a very different impact on the drift of pH of the formulation when in contact with the primary container, suggesting that the quantity and strength of the pH buffer possibly used in the formulation will have to be different between a vial and e.g. a prefilled syringe.

Silicone oil is also known to cause the appearance of dropplets or "particles", of all sizes, especially with biotech formulations: the trend, in these formulations, is towards higher and higher concentration in the active ingredients - in particular to reduce the number of injection per week or month and improve patient convenience and adherence. In many instances, the drug is concentrated up to a level where its solubility is at risk. In order to avoid re-precipitation, massive amounts of surfactants are included in the formulation. When in contact with the silicone oil of syringes or cartridges, these surfactants emulsify the silicone oil, producing silicone oil droplets of varying size in the formulation. These silicone oil droplets are invisible below 100 micrometers, but it is now well established that silicone oil droplets of a size smaller than 100 micrometer may eventually coalesce to larger size droplets, and the final production of visible particles, taking the form of a turbidity in the container which makes the dose unsuitable for medical use.

Another significant difference lies in the glass delamination propensity: glass vials undergo much higher temperatures - especially the bottom of the vial - than syringes or cartridges. This is because the forming of vials requires to cut the glass tube to form the bottom of the vial, and this requires a much higher thermal budget than the forming of the tip in syringes or of the collar in cartridges. One known consequence of this higher temperature budget is the partial demixion of non miscible phases of the glass, and the sub-surface depletion in alkaline ions such as Na⁺. It is also known that when a glass having undergone such modifications is re-hydrated in contact with a water based formulation, sub-surface stress is abruptly modified, provoking cracks that eventually lead to the formation of glass flakes detaching from the surface as glass particles. The extent of delamination is further dependent upon e.g. the pH of the formulation, as well as of the presence of complexing agents like citric or oxalic acid. Since 2010, there has been ca. 12 recalls of marketed drugs due to the detection of glass particles in vials stemming from delamination.

In this regard, an example of a document relating to this problem is "Pharmaceutical Packaging" of Michael Bonewitz (31 December 2008, XP055196222).

Last but not least, each and every part of the surface of any container may release chemical compounds when in contact with the pharmaceutical formulation. In order to anticipate which types of compounds are "releasable", primary container manufacturers often perform a study of "extractables": they use a variety of solvents and experimental conditions to extract any chemical that can be extracted from the material of the primary container surface, and determine the molecular structure of said chemical, to possibly find its source and eliminate it if needed. When receiving the primary containers, pharmaceutical companies perform a study of "leachables", i.e. of the compounds that are actually extracted when their sole drug formulation is put into contact with the primary container. In principle, leachables are in the list of extractables, but the reverse is not true.

Being made according to different processes, sometimes on the basis of different materials, vials on the one hand, and syringes or cartridges on the other hand, rubber caps on the one hand, and syringe or cartridge plungers on the other hand, may have different extracables and/or leachables.

This non exhaustive list of differences between vials on the one hand, and syringes or cartridges on the other hand, illustrate the reason why instabilities may appear when changing the primary container between Phase 2 and Phase 3: due to the interaction between drug formulations and containers, one may consider that primary containers are merely another - non liquid - additive in the formulation: changing the primary container at the end of Phase 2 amounts to changing one additive in the formulation. As this formulation was optimized through a DOE comprising a given list of additives, changing one means that the liquid formulation is not at its optimal stability composition.

That is why after transition from a primary glass container to another primary glass container having a different material composition, a re-optimization of the drug formulation is required to ensure drug formulation stability in the last used primary glass container.

Solving the re-optimization of the drug formulation of course involves costs and significant and lengthy rework. Shelf life of prefilled drug formulations of 1 year - and sometimes more - are frequent. This means that the stability of the formulation optimized in the vial has to be evaluated in the syringe or cartridge for this period of time before any conclusion can be drawn as to the need for reformulation. In the best of cases, an accelerated aging protocol can be designed which gives this information in a shorter amount of time, but the final validation of the stability will have to be made in real time for regulatory approval. More and more, drug development teams are trying to anticipate this overhead time by studying formulation stability as early as Phase 1 or during Phase 2: while this is gaining some time, this does not change the extra effort for reformulation and need to re-demonstrate stability of the new formulation in the prefilled container, as well as the extra costs associated to this reformulation, be it done in parallel or not. All in all, it is estimated that vial to prefill transition takes between 12 and 24 months, and costs between 500,000 and 3 million Euros.

In some instances, the drug formulation turns out to be so sensitive to the new materials it is exposed to when transitioning from vial to a prefilled format that no satisfactory solution is found: efforts to transition to a prefilled container are finally abandoned, and the drug formulation enters Phase 3 - and is then marketed if ever marketed - in vial.

There is no systematic solution to this issue of vial to prefill transition. It is the objective of the present invention to overcome drawbacks of the prior art.

Within this technical problem, a task of the present invention is to provide a method of handling a prefilled formulation which guarantees stability of the drug formulation with no - or very limited - need of further re optimization of the drug formulation when transitioning from vials to prefilled formats.

Another task of the present invention is to provide a method of handling a prefilled formulation preventing denaturation of the protein drug formulation.

These tasks are solved by the present invention providing a method of handling of a liquid drug formulation, comprising a step of providing a first primary container and coating the inner surface of the first primary container to be exposed to the formulation with a barrier layer to prevent or at least limiting any escape of material from the inner surface of the first primary container, a step of providing a second primary container and coating the inner surface of the second primary container to be exposed to the formulation with a barrier layer to prevent or at least limit any escape of material from the inner surface of the second primary container, wherein the barrier layer coating the inner surface of the second primary container has the same material composition as the barrier layer coating the inner surface of the first primary container, and a step of transition from handling the formulation filled in the first primary container at a first handling phase to handling the formulation filled in the second primary container at a subsequent handling phase so as the formulation exhibits with the second primary container the same kind of interaction as with the first primary container.

The barrier layer entirely covers the inner surface of the primary container.

Thanks to a coating with a barrier layer having the same material composition applied to the inner surface of primary containers, the level of contaminant particles in the drug formulation is eliminated or at least significantly reduced and the protein formulation stability can be secured as well during the entire validation process of a compound to become an approved drug.

With the present invention, as the barrier layer acting as an "additive" to the formulation of the drug is always the same for all glass primary containers used during the drug validation process, a drug formulation showing stability in a vial being one primary glass container used in an early step of the drug validation process will show stability as well in a prefilled type container - syringe or cartridge - in a following step of the drug validation process.

The proposal is therefore to use a vial, preferably a glass vial, having the inner surface coated with such a barrier layer as a primary container when the initial Design Of Experiment (DOE) is performed in research level formulation optimization, then keep such a vial as a primary container until the end of phase 2, then let transition from phase 2 to phase 3 occur with substitution of such a vial with a cartridge or syringe serving as a primary container for the drug and having an inner surface coated with a barrier layer having the same material composition as the one coating the inner surface of such a vial.

More specifically, the compatibility of the formulation with the materials of the containers of the invention is secured upfront at the research level during the DOE: this step is being carried out anyway, to produce the formulations that will undergo the various trials, so the use of these coated containers does not add any extra delay in the drug development process. Let's imagine that the drug has successfully passed Phase 1 and Phase 2: when the time comes to move it to a prefilled cartridge or syringe to enter Phase 3, it is expected that the drug formulation will be as stable in the prefilled format as it was in the vial format, since the materials it is "seeing" are the same as in the vial. For regulatory purposes, this needs to be validated explicitly, and a stability study will have to be performed in the new containers, but this can then be done in parallel to Phase 2. It cannot be excluded, however, that the stability in the new containers could be assessed by equivalence, thus bypassing the explicit time and costs of the stability assessment.

As a result, some 12 to 24 months could be gained in the kick-off of Phase 3, and in case of a favorable outcome, this means that the drug can be launched to the market 12 to 24 months earlier than without the present invention, with no risk of failing in the vial-to-prefilled transition. This also means 12 to 24 more months of on patent revenues for the pharmaceutical company: it is estimated that there are today ca. 9000 biotech drugs in the pipeline in Phases 2 and before. About a third of these drugs (3,200) are expected to be formulated for a prefilled format in the end, and finally about 435 will eventually reach the market successfully (14%). Considering a hypothetical adoption of the solution of the invention for these drugs, and considering that it enables a launch 1 year earlier than without this solution, the gain for the pharmaceutical industry in advancing its revenues has a Net Present Value of $4 billions (at a discount rate of 9%) over the lifetime of these pipeline drugs. This is pure value creation, as it does not require any extra drug development work.

Since the inner surface of the assembly comprising the primary container and the respective cap to be exposed to the prefilled formulation is usually composite - containing e.g. glass and rubber parts - it may be that several types of barrier layers are needed to coat said surface in a way that the same overall surface is exposed to the drug formulation. It should also be noted that different barrier layers may be applied on different parts of said inner surface, depending on the nature of the materials to bring barrier to. For example, it may be that a first barrier layer is applied to the glass part of the inside of a vial, while a second - different - barrier layer is applied to the part of the rubber cap surface to be exposed to the drug formulation.

Finally, it should be noted that since the added layers of the invention are intended to have a barrier effect vs the extraction of substances from the bulk of the various primary containers, and to expose to the formulation a surface that will be similar whatever the surface or container underneath, the scope of the invention is not restricted to glass primary containers. It can be applied, for example, to plastic containers.

These and other features of the present invention will be more clear after consideration of the following embodiments. According to the invention, the first primary container has at least a different shape and/or has a different material composition than the second primary container.

According to a preferred embodiment of the invention the second primary container material composition comprises a silicone oil lubricant.

According to a preferred embodiment of the invention the first primary container is a vial provided with a cap.

According to a preferred embodiment of the invention the second primary container is a barrel of a prefillable syringe having a plunger with inner cone surface.

According to a preferred embodiment of the invention the second primary container is a barrel of a prefillable cartridge having a plunger with inner cone surface.

According to the invention the barrier layer is applied by first applying a layer of adhesion primer, then a layer of silicone oil on all or part of the inner surface of the prefillable container, then by treating the coated surfaces with an argon plasma to achieve crosslinking of part of the silicone oil layer.

According to a preferred embodiment of the invention the barrier layer is applied by chemical vapor deposition (CVD) or atomic layer deposition (ALD) on all or part of the inner surface of the prefillable container.

According to the invention all or part of the inner surface of the primary container has a coating comprising a barrier layer made of crosslinked silicone.

The invention is now more clearly explained in the hereinbelow examples which make reference to following figures:
Figure 1 shows the coating process of example 1;
Figure 2 shows the coating of the pre-treatment layer and of the silicone oil layer in a syringe of example 1;
Figure 3 shows a graph gathering results of analysis of the number of particles contained in the WFI of example 3;
Figure 4 shows a graph illustrating the barrier effect of the coating of example 3;
Figure 5 shows a graph illustrating CD spectra of coated vials and coated syringes for each protein of example 3;
Figure 6 shows the plant to perform the coating process of example 4.

### EXAMPLES

### EXAMPLE 1: Process of application of a plasma treated silicone layer onto a syringe and vial.

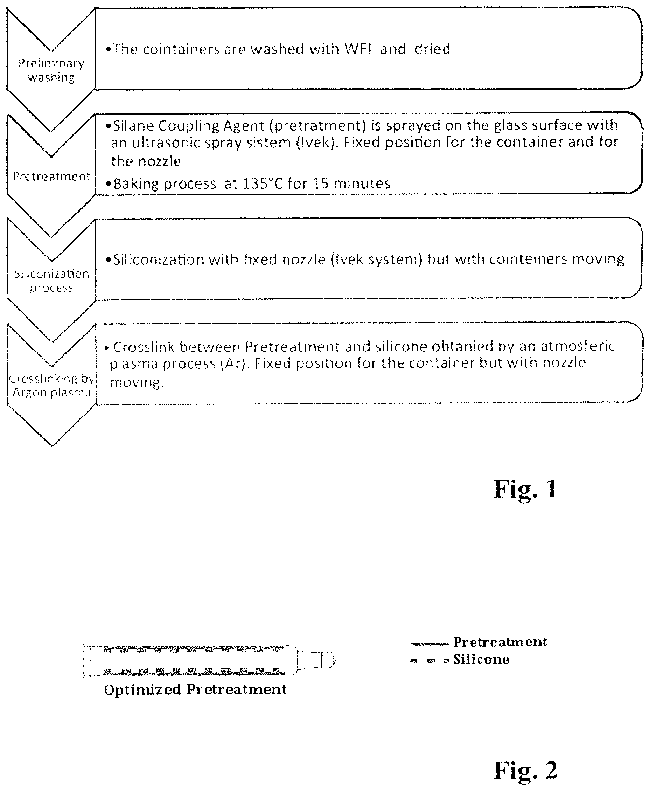

The overall process used to coat the inner surface of a container is described in Fig. 1.

The adhesion primer is applied onto the glass surface by uniformly spraying a 2% w/w solution of [(Bicycloheptenyl)ethyl] trimethoxy-silane in isopropanol inside the surface of the primary container. This can be done using a sampleholder in which the nozzle is fixed, and the primary container can be moved relatively to the nozzle, in order to spray on the whole inner surface of the primary container by moving the primary container as the spray is on. A volume of 10 microliters is usually enough to coat the inner surface of a 1 mL syringe, at a flow rate of 10 microliters per second.

As a second step, the surface is then coated, still by spray, with Dow Corning 360 (1000 cts) silicone oil. This is done with a similar setup in which the nozzle can move relative to the container at a speed of 55 mm/s. The temperature of the flow controller we have used was 25 psi and pressure of the tank 9 psi. The temperature of the nozzle was 150°F (65.56°C), and the spraying time 1 second, enabling a uniform silicone inside the whole of the container.

As a third and final step, a plasma treatment is applied: the parameters of the plasma treatment are listed in the table below.

**Plasma Parameters:**

| **parameter** | **value** | **note** |
|---|---|---|
| Input voltage | 115-240VAC, 50-60Hz | |
| Max fuse protection | 3A | |
| Maximum Power output | 100 W | |
| Gas Feed-in pressure | 50-60 psi | |
| Argon gas input | 10-13 SLM | Minimum grade is 99,9% pure |
| Argon gas setting | 4-6 SLM | |
| Relative Humidity | 10-70% | |
| Duty cycle (plasma) | up to 50% for 3 sec max on time | |
| Ambient temperature | 5-35°C | |
| Treatment time | | depend on the test to carried out (best 0,5sec) |
| Nozzle insertion depth from bottom | 5-10 mm | for syringes sticked needle. in this case the luer vent is blocked |

The table below shows the typical plasma times to treat the silicone layer of a 1 mL syringe:

| **Category** | | **Pretreatment** | **Silicone quantity** | **Plasma time (sec)** |
|---|---|---|---|---|
| OMPI Standard EZ-FILL™ | | NO | 0.7 (=0,5 mg) | NO |
| TL | TL1 | Optimized | 0,4 | 0,1 |
| | TL2 | Optimized | 0,4 | 0,2 |
| | TL3 | Optimized | 0,4 | 0,3 |
| | TL4 | Optimized | 0,4 | 0,5 |

The table below shows the thickness - measured using rapID - of the resulting layer after applying the EP (European Pharmacopeia) autoclave protocol:

**Rap-id RESULTS (autoclave method):**

| **Category** | | **Thickness (nm)** | |
|---|---|---|---|
| | | Before Autoclave | After Autoclave |
| OMPI Standard EZ-FILL™ | | 359 | 133 |
| TL | TL1 | 152 | 89 |
| | TL2 | 179 | 99 |
| | TL3 | 213 | 111 |
| | TL4 | 241 | 104 |

The following table shows the resulting number of particles - essentially silicone oil droplets - as a function of particle size, as measured by a Micro Flow Imaging Series 5200 tool (MFI), after EP autoclave:

**MFI RESULTS (autoclave method):**

| **Category** | | **Total particles** | | | | |
|---|---|---|---|---|---|---|
| | | 1-2 µm | 2-5 µm | 5-10 µm | 10-25 µm | 25-50 µm |
| OMPI Standard EZ-FILL™ | | 155442 | 100199 | 15399 | 429 | 4 |
| TL | TL1 | 2800 | 1278 | 152 | 19 | 3 |
| | TL2 | 3164 | 1136 | 138 | 16 | 2 |
| | TL3 | 2387 | 838 | 91 | 10 | 1 |
| | TL4 | 2243 | 1205 | 122 | 26 | 5 |

As can be seen, the number of particles of all sizes is significantly reduced compared to the reference syringe.

### EXAMPLE 2: Process of building of the barrier layer onto the surface of plungers

Rubber components received from suppliers already contain a silicone layer (in order to facilitate demolding and separation of said plungers). They are treated directly with argon plasma with parameters similar to those of example 1:

| **Plunger (without syringe)** | **Description** | **Total Particles** | | | | | **GLIDING (EZ-FILL™ method)*** | |
|---|---|---|---|---|---|---|---|---|
| | | 1-2 µm | 2-5 µm | 5-10 µm | 10-25 µm | 25-50 µm | Breaking L (N) | Gliding L (N) |
| X | Blank (filtered water) | 527 | 243 | 26 | 5 | 0 | X | X |
| Datwyler (N=5 for each category) | No plasma | 4555 | 2090 | 154 | 2 | 0 | 2,19 | 1,90 |
| | Plasma 0,5 sec | 2929 | 724 | 41 | 2 | 0 | 2,22 | 1,93 |
| | Plasma 2,0 sec | 2472 | 1003 | 56 | 2 | 0 | 1,95 | 1,57 |
| | Plasma 4,0 sec (2 times the plasma 2,0 sec) | 2266 | 1116 | 41 | 2 | 0 | 2,38 | 1,88 |
| West (n=5 for each category) | No plasma | 9004 | 2173 | 1459 | 48 | 0 | 1,41 | 1,26 |
| | Plasma 0,5 sec | 6520 | 3417 | 1067 | 15 | 0 | 1,53 | 1,16 |
| | Plasma 2,0 sec | 4529 | 2958 | 283 | 8 | 0 | 1,59 | 1,28 |
| | Plasma 4,0 sec (2 times the plasma 2,0 sec) | 5274 | 3061 | 218 | 1 | 0 | 1,72 | 1,05 |

These plungers are then tested in syringes coated as described in example 1. The above table gathers the breaklose force and maximum gliding force in each case, as well as particle count after autoclave.

The autoclave protocol used herein consists in:
1. Filling a particle-free tube with 3.9 mL of filtered water and put 3 plunger inside (for the syringes the filling is 1.3 mL for each syringe)
2. Cover the tube with aluminum foil
3. E.P. autoclaving cycle
4. Rinse the sample before measure with MFI

The above table shows the considerable reduction in particle count brought by the plasma treated silicone layer, hence illustrating the barrier layer, while keeping the good gliding properties of the plunger in the syringe.

### EXAMPLE 3: Comparison of a vial and a syringe to which the same barrier layer is applied

The barrels of a glass vial and a glass syringe - made from the same glass type and quality - are treated according to Example 1.

Rubber caps and plungers - made from the same rubber type and quality - are treated according to Example 2.

The vial and syringe are filled with Water For Injection (WFI), assembled with their respective rubber part, and the European Pharmacopeia autoclave protocol is applied to each.

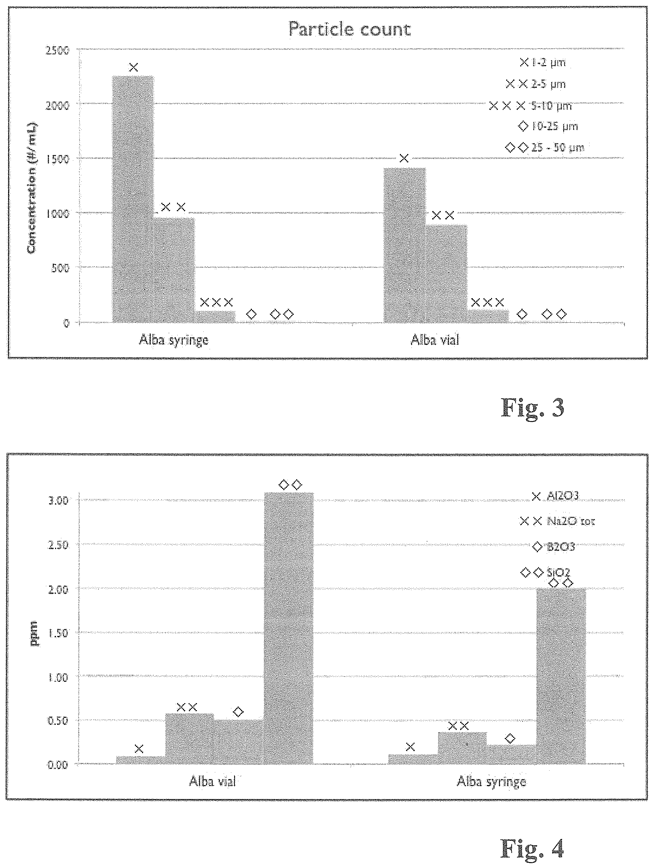

The number of particles contained in the WFI is analyzed using the MFI tool. The graph of Fig. 3 gathers the results obtained.

It shows that although the vial and syringe are made through different process, they have very similar particle distribution profiles. One shall note that the difference for particle sizes of 1-2-microns is actually within deviation of the analytical method for measurements that were not carried out in clean room.

The solutions contained in the respective containers are also analyzed by ICP-OES (Induced Coupled Plasma Optical Emission Spectroscopy, Varian 710) to determine the various chemical elements released by the full surface in contact with the water. The graph below is showing the results presented according to European Pharmacopeia guidelines.

The graph of Fig. 4 shows that both the coated vial and the coated syringe have a very low level in all elements, illustrating the barrier effect of the coating. One shall note in particular the level of Na2O, well below the EP threshold of 3.20 ppm, suggesting that these containers will have low impact on the pH of the formulation they will contain. A direct measurement of the EP value - i.e. the volume, in mL, of 0.1N hydrochloric acid needed to re-adjust a pH = 6.8 solution put in contact with the container, and which is a direct indication of the ion exchange at the top surface - gives EP = 0.3 for both the coated vial and the coated syringe: this is a very low value, well below the threshold of 1.30 of the European Pharmacopeia.

In the absence of complexing agents in the formulation, such a low EP value is also indicative of a very low propensity for glass delamination, which is further illustrated by the SiO2 values: despite the presence of the crosslinked silicone oil layer in both containers: in the case of the vial, this means that glass delamination is also very low, thanks to the barrier effect of the coating, as most of the SiO2 measured by ICP-OES in vials is usually due to glass delamination. As a comparison, SiO2 levels as measured by ICP-OES using the same protocol are indicated in the table below:

| | SiO2 (ppm) |
|---|---|
| Coated syringes | 2,00 |
| Siliconized syringes | 12,00 |
| Bulk syringes | 22,00 |
| | |
| | |
| | |

| | SiO2 (ppm) |
|---|---|
| Coated vials | 3,00 |
| Bulk vials | 9,00 |

These results show how much the present invention is bringing vials and syringes to similar and very low levels of SiO2 release, although the original glass containers have very different and high values.

Finally, we have tested the impact of the adsorption of two proteins onto the surface of the coated vial and of the coated syringe, on the conformational change they undergo. In this way, we can probe the stability of the proteins in their prefilled formulation.

For this purpose, we have used lysozyme and α-bovine lactalbumine: these two proteins have comparable molecular mass but tend to behave differently, lysozyme adsorbing on all surfaces, while α-bovine lactalbumine essentially adsorbs onto hydrophobic surfaces:

| | α-lactalbumin 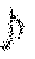 | Lysozyme 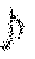 |
|---|---|---|
| Sequence lenght | 123 AA | 129 AA |
| Molecular weight | 14,175 Da | 14,307 Da |
| Isoelectric point | 4.5 | 11.35 |
| *α* Helix | 74% | 60% |
| *β* Strand | 16% | 20% |

All the wavelenght scan measurement were conducted at 25°C using 1.0 x 0.1 cm quartz cuvettes for far-UV CD (circular dichroism) and 0.5 x 1.0 cm quarz cuvettes for near-UV CD. Far-UV circular dichroism spectra were taken from 210 to 250 nm at 20 nm/min with 16 s response time using 0.1 mg/mL solutions of samples. Near-UV circular dichroism spectra were taken from 320 to 250 nm at 20 nm/min with a 16 s response time using the protein solution picked up from the vials incubated. The acquired spectra were normalized for concentration determined empirically based on the absorbance and mass of the protein.

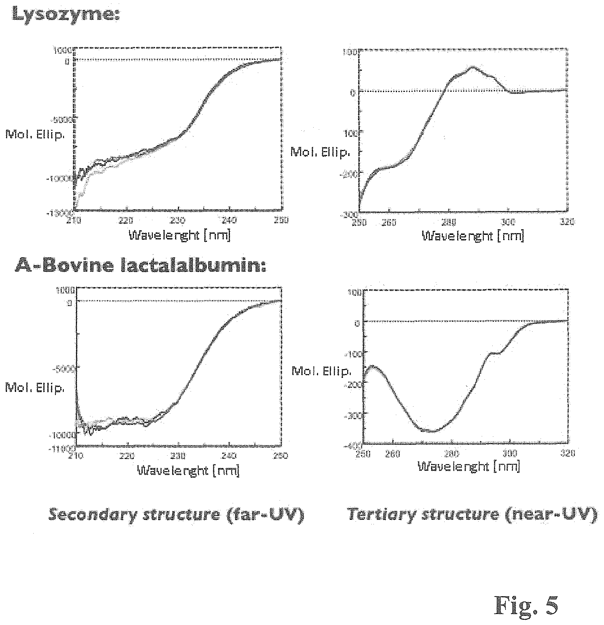

In Fig. 5, CD spectra of coated vials and coated syringes have been displayed in the same graph for each protein, in the two wavelength ranges, after 7 days of incubation at 40°C.

The striking result from curves of Fig. 5 is that they are all exactly superimposed, showing that no difference can be evidenced in conformational change of the proteins when they are in a coated vial vs when they are in a coated syringe.

Overall, these results show that the plasma treated silicone layer applied to the barrel of a vial in addition to that of a syringe the plasma treatment of rubber parts used to close these respective containers build inner surfaces that have very similar behaviors in terms of glass delamination, particle level, extractables and pH drift propensity, although on dissimilar containers.

### EXAMPLE 4 (not according to the invention):

MOCVD SiO₂ deposition on vials Metal Organic Chemical Vapour Deposition of SiO₂ has been carried out on vials.

### Reagents:

The precursor is [Tris (dimethylamino)]silane ((CH₃)₂N)₃SiH (from STREM chemical, also Aldrich)
The gases used are anhydrous N₂ and O₂ :
- N₂ enters in the furnace or directly through a bypass or during the growth through the precursor bubbler. It pass over the liquid surface and not bubbling inside it.
- O₂ enters in the furnace through a water bubbler. The gas pass over the liquid surface and not bubbling inside it.

### Fluxes

- N₂ 50 sccm
- O₂ 150 sccm

### Temperatures

- The precursor was thermostated at 30°C and also the subsequent transport lines.
- The water was thermostated at 35°C and also the subsequent transport lines.
- The furnace was regulated at 570°C.

### Pressure

- atmospheric

### Time

- 50" pre deposition
- 2' and 30" deposition time

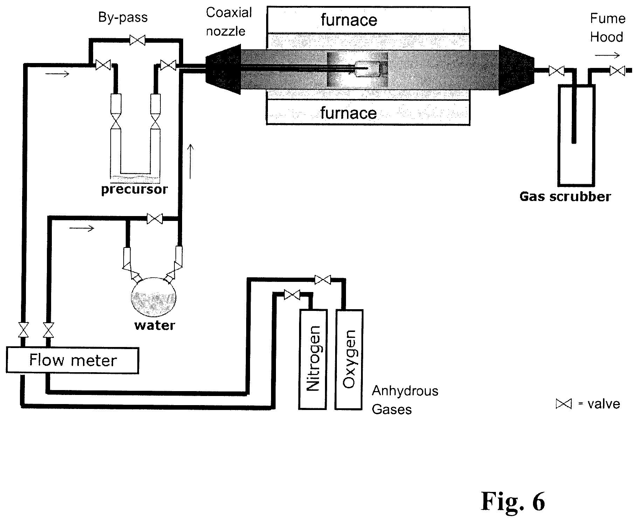

There were not a surface preparation. The vial was inserted progressively in 15 minutes inside the furnace (to avoid thermal shock). Both gases were opened for 50 seconds to have an homogenous atmosphere of nitrogen and wet-oxygen inside the vials. Then the precursor bubbler was opened and the bypass closed for 2 minutes and 30 seconds (the water bubbler is always open.) At the end of the deposition the bubbler valves were closed and subsequently also both the gas. The vial was then extracted from the furnace in 15 minutes to avoid thermal shocks. There were no post deposition treatments. The plant to perform this process is shown in Fig. 6.

This example describes the coating process for a vial, but it is applicable the same way for a syringe or cartridge, and hence in this way it can be built a coating that is the same in all containers.

## Claims

1. Method of handling of a liquid drug formulation, comprising a step of providing a first primary container and coating the inner surface of the first primary container to be exposed to the formulation with a barrier layer to prevent or at least limiting any escape of material from the inner surface of the first primary container, a step of providing a second primary container different than the first primary container and coating the inner surface of the second primary container to be exposed to the formulation with a barrier layer to prevent or at least limit any escape of material from the inner surface of the second primary container, wherein the barrier layer coating the inner surface of the second primary container has the same material composition as the barrier layer coating the inner surface of the first primary container, and a step of transition from handling the formulation filled in the first primary container at a first handling phase to handling the formulation filled in the second primary container at a subsequent handling phase so as the formulation exhibits with the second primary container the same kind of interaction as with the first primary container; wherein the first primary container and the second primary container have a respective closure element whose inner surface to be exposed to the formulation is coated with a barrier layer having the same material composition and wherein the first primary container has different shape and/or different material composition than the second primary container,
wherein the barrier layer is applied by applying on the inner surface of the primary container a layer of adhesion primer, then a layer of silicone oil on the layer of adhesion primer, then by treating the coated inner surface with an argon plasma to achieve crosslinking of part of the silicone oil layer.

2. Method of handling a liquid drug formulation according to claim 1, wherein the barrier layer coating the first and second primary container is either different or the same as the barrier layer coating the corresponding closure element.

3. Method of handling a liquid drug formulation according to claim 1, wherein the first primary container has different shape and same material composition than the second primary container, the first and second primary containers are made of glass and the corresponding closure elements are made of rubber.

4. Method of handling a liquid drug formulation according to claim 1, wherein the barrier layer is applied by chemical vapor deposition (CVD) or atomic layer deposition (ALD) on the inner surface of the primary container.

## Patentansprüche

1. Verfahren zur Handhabung einer flüssigen Arzneimittelformulierung, umfassend einen Schritt des Bereitstellens eines ersten Primärbehälters und des Beschichtens der Innenfläche des ersten Primärbehälters, die der Formulierung ausgesetzt werden soll, mit einer Sperrschicht, um jedwedes Entweichen von Material aus der Innenfläche des ersten Primärbehälters zu verhindern oder zumindest zu begrenzen, einen Schritt des Bereitstellens eines zweiten Primärbehälters, der sich von dem ersten Primärbehälter unterscheidet, und des Beschichtens der Innenfläche des zweiten Primärbehälters, die der Formulierung ausgesetzt werden soll, mit einer Sperrschicht, um jedwedes Entweichen von Material aus der Innenfläche des zweiten Primärbehälters zu verhindern oder zumindest zu begrenzen, wobei die Sperrschicht, die die Innenfläche des zweiten Primärbehälters beschichtet, die gleiche Materialzusammensetzung aufweist wie die Sperrschicht, die die Innenfläche des ersten Primärbehälters beschichtet, und einen Schritt des Übergangs von der Handhabung der Formulierung, gefüllt in den ersten Primärbehälter, in einer ersten Handhabungsphase zur Handhabung der Formulierung, gefüllt in den zweiten Primärbehälter, in einer nachfolgenden Handhabungsphase, so dass die Formulierung mit dem zweiten Primärbehälter die gleiche Art der Interaktion wie mit dem ersten Primärbehälter aufweist; wobei der erste Primärbehälter und der zweite Primärbehälter ein entsprechendes Verschlusselement aufweisen, dessen Innenfläche, die der Formulierung ausgesetzt werden soll, mit einer Sperrschicht mit der gleichen Materialzusammensetzung beschichtet ist, und wobei der erste Primärbehälter eine andere Form und/oder eine andere Materialzusammensetzung als der zweite Primärbehälter aufweist,
wobei die Sperrschicht aufgebracht wird durch das Aufbringen einer Schicht Haftprimer auf die Innenfläche des Primärbehälters, dann einer Schicht Silikonöl auf die Schicht Haftprimer, dann durch das Behandeln der beschichteten Innenfläche mit einem Argonplasma, um eine Vernetzung eines Teils der Silikonölschicht zu erreichen.

2. Verfahren zur Handhabung einer flüssigen Arzneimittelformulierung nach Anspruch 1, wobei die Sperrschicht, die den ersten und zweiten Primärbehälter beschichtet, entweder unterschiedlich oder gleich der Sperrschicht, die das entsprechende Verschlusselement beschichtet, ist.

3. Verfahren zur Handhabung einer flüssigen Arzneimittelformulierung nach Anspruch 1, wobei der erste Primärbehälter eine andere Form und die gleiche Materialzusammensetzung als der zweite Primärbehälter aufweist, der erste und zweite Primärbehälter aus Glas sind und die entsprechenden Verschlusselemente aus Gummi sind.

4. Verfahren zur Handhabung einer flüssigen Arzneimittelformulierung nach Anspruch 1, wobei die Sperrschicht durch chemische Gasphasenabscheidung (CVD) oder Atomlagenabscheidung (ALD) auf die Innenfläche des Primärbehälters aufgebracht wird.

## Revendications

1. Procédé de manipulation d'une formulation de médicament liquide, comprenant une étape de fourniture d'un premier récipient principal et de revêtement de la surface interne du premier récipient principal à exposer à la formulation avec une couche barrière pour empêcher ou au moins limiter une quelconque fuite de matériau de la surface interne du premier récipient principal, une étape de fourniture d'un second récipient principal différent du premier récipient principal et de revêtement de la surface interne du second récipient principal à exposer à la formulation avec une couche barrière pour empêcher ou au moins limiter une quelconque fuite de matériau de la surface interne du second récipient principal, dans lequel la couche barrière revêtant la surface interne du second récipient principal a la même composition de matériau que la couche barrière revêtant la surface interne du premier récipient principal, et une étape de transition de la manipulation de la formulation versée dans le premier récipient principal lors d'une première phase de manipulation à la manipulation de la formulation versée dans le second récipient principal lors d'une phase de manipulation consécutive de façon à ce que la formulation présente avec le second récipient principal le même type d'interaction qu'avec le premier récipient principal ; dans lequel le premier récipient principal et le second récipient principal ont un élément de fermeture respectif dont la surface interne à exposer à la formulation est revêtue d'une couche barrière ayant la même composition de matériau et dans lequel le premier récipient principal a une forme différente et/ou une composition de matériau différente du second récipient principal,
dans lequel la couche barrière est appliquée en appliquant sur la surface interne du récipient principal une couche d'apprêt d'adhésion, puis une couche d'huile de silicone sur la couche d'apprêt d'adhésion, puis en traitant la surface interne revêtue avec un plasma d'argon pour obtenir la réticulation d'une partie de la couche d'huile de silicone.

2. Procédé de manipulation d'une formulation de médicament liquide selon la revendication 1, dans lequel la couche barrière revêtant les premier et second récipients principaux est soit différente soit identique à la couche barrière revêtant l'élément de fermeture correspondant.

3. Procédé de manipulation d'une formulation de médicament liquide selon la revendication 1, dans lequel le premier récipient principal a une forme différente et une composition de matériau identique au second récipient principal, les premier et second récipients principaux sont en verre et les éléments de fermeture correspondants sont en caoutchouc.

4. Procédé de manipulation d'une formulation de médicament liquide selon la revendication 1, dans lequel la couche barrière est appliquée par dépôt chimique en phase vapeur (CVD) ou dépôt de couches atomiques (ALD) sur la surface interne du récipient principal.
